# EUROPEAN PATENT SPECIFICATION

(11) **EP 1 474 154 B1**
(45) Date of publication and mention of the grant of the patent: **15.06.2005**
(21) Application number: 03702746.3
(22) Date of filing: 07.02.2003
(51) Int. Cl.: A61K 31/734, A61K 9/00, A61K 9/48, A61P 1/04

(54) **IMPROVEMENTS IN OR RELATING TO COMPOSITIONS**
VERBESSERUNGEN AN ODER IN BEZUG AUF ARZNEIZUSAMMENSETZUNGEN
AMELIORATIONS APPORTEES A DES COMPOSITIONS OU LIEES A CES COMPOSITIONS

(30) Priority: 12.02.2002 GB 0203269
(43) Date of publication of application: 10.11.2004
(73) Proprietor: Reckitt Benckiser Healthcare (UK) Limited, Slough, Berkshire SL1 3UH (GB)
(72) Inventor: AXFORD, Sophie, Eugenie, Bicester Oxfordshire OX26 6SR (GB); DICKSON, Paul, Andrew, Hull HU8 7DS (GB); JOLLIFFE, Ian, Gordon, Hull HU8 7DS (GB); MYRVOLD, Rolf, N-1389 Heggedal (NO); MARSHALL, Paul, Ashby-de-la-Zouch Leicestershire LE65 2N (GB)
(74) Representative: Brown, Andrew Stephen
(86) International application number: PCT/GB2003/000593
(87) International publication number: WO 2003/068246

(56) References cited:
- WO-A-95/11668
- WO-A-96/27368
- US-A- 4 140 760

## Description

The present invention relates to pharmaceutical compositions, and in particular to composition for the treatment of reflux oesophagitis, gastritis, dyspepsia or peptic ulceration or for use as a sustained releasing or targeted delivery compositions.

Reflux oesophagitis occurs when small amounts of gastric juice, food and/or bile acids pass into the lower part of the oesophagus and cause oesophageal inflammation accompanied by pain which may manifest itself in the form of heartburn.

One approach to the problem of reflux oesophagitis has been to administer a preparation which on contact with gastric acid generates a carbonated gelatinous foam or raft which floats on the stomach contents. When reflux occurs it is this raft which precedes the stomach contents into the oesophagus, thus protecting the mucosa from further irritation. Known preparations of this type include liquid preparations comprising sodium alginate, sodium or potassium bicarbonate and calcium carbonate. Such compositions are sold under the trade marks GAVISCON and GAVISCON ADVANCE and are described in GB-A-1,524,740 and WO 95/11668.

Other such preparations are those in solid form, for example in the form of powders or tablets, such as those which again are sold under the trade mark GAVISCON. Such preparations comprise alginic acid, sodium bicarbonate and calcium carbonate. The alginic acid and the bicarbonate and carbonate react in the aqueous environment of the mouth to form an alginate foam, which is then swallowed. In the acidic stomach environment the alginate is converted back into insoluble alginic acid, which then forms the raft on top of the stomach contents.

It has been found that solid compositions which foam in the mouth are difficult, and sometimes unpleasant, to swallow. In order to provide a non-foaming, solid composition we have tried to replace the alginic acid by an alginate. However, we have found that such compositions have extremely poor mouth feel. The alginate is sticky and causes the composition to stick to the palate, and especially to the teeth.

We have surprisingly found that the mouth feel and stickiness of such compositions can be improved by including a further component in the composition.

Accordingly the present invention provides a solid, ingestible composition comprising:
a. an alginate;
b. a bicarbonate and/or carbonate; and
c. a C₂-C₅ polyol or poly(C₂-C₅ alkylene glycol) having a molecular weight of at least 6000.

The composition of the present invention has less foaming than the tablets currently sold under the trade mark GAVISCON since it comprises an alginate rather than alginic acid. It also has a good mouth feel and does not stick to the teeth as much as a composition which does not comprise a polyol or polyalkylene glycol.

The composition of the present invention comprises an alginate. Any alginate may be used, but it is especially desirable to use an alkali metal salt of an alginate, such as sodium or potassium alginate. Preferably a low viscosity grade of the alginate is used. These are generally grades of alginate for which the viscosity of a 10% weight/volume aqueous solution, when determined on a Brookfield RVT viscometer using spindle number 3 at 20 r.p.m. at 20 °C, falls within the range of 200 to 1,500 mPa.s. An example of a suitable commercial grade of low viscosity sodium alginate is Protanal LFR 5/60, obtainable from FMC BioPolymer. High viscosity grades of alginate may also be used. These are generally grades alginate for which the viscosity of a 1% weight/volume aqueous solution, when determined on a Brookfield RVT viscometer using spindle number 3 at 20 r.p.m. at 20 °C, is above 500 mPa.s. An example of a suitable commercial grade of high viscosity sodium alginate is Protanal SF200, also obtainable from FMC BioPolymer

The compositions of the present invention generally have a content of alginate of from 2 to 90 wt%, preferably 5 to 50 wt%, based on the total weight of the composition.

The compositions of the present invention also comprise a bicarbonate and/or carbonate. Examples of bicarbonates are alkali metal bicarbonates such as sodium and potassium bicarbonate and alkaline earth metal bicarbonates. One or two or more different bicarbonates may be used. Examples of carbonates are alkali metal carbonates such as sodium and potassium carbonate and alkaline earth metal carbonates such as calcium and magnesium carbonate. Further examples are aluminium carbonate and mixed alkali metal carbonates such as sodium glycine carbonate. One or two or more different carbonates may be used. Furthermore one or more bicarbonates may be used with one or more carbonates. Especially preferred combinations are sodium and/or potassium bicarbonate and calcium carbonate.

The carbonate and/or bicarbonate are present in amounts such that they provide an adequate volume of gas (carbon dioxide) to float the gel produced when the alginate contacts the gastric acid in the stomach. The rigidity and thickness of the carbonate raft will depend, for example, upon the relative amounts of carbonate and/or bicarbonate and on the grade of the alginate.

If used alone, the bicarbonate is generally present in the compositions of the present invention in an amount of from 1.5 to 35 wt%, preferably 2 to 15 wt%, most preferably 3 to 10 wt%. If used alone, the carbonate is generally present in the compositions of the present invention in an amount of from 0.2 to 55 wt%, preferably 0.5 to 10 wt%, most preferably 1 to 4 wt%.

Preferably the bicarbonate and carbonate may also be present together in the composition, preferably from 1 to 20 wt%, for example in a total amount of from 1 to 40 wt%, preferably 1 to 12 wt%. Approximately equal amounts of the bicarbonate and carbonate may be present in the composition. Alternatively, the composition may comprise more bicarbonate than carbonate. The weight ratio of bicarbonate to carbonate in the composition may be from 1:1 to 2:1.

The compositions of the present invention also comprise a C₂-C₅ polyol or poly(C₂-C₅ alkylene glycol). Suitable polyols have 2, 3, 4 or 5 carbon atoms and contain 2 or more hydroxy groups, for example 2, 3, 4 or 5 hydroxy groups. Examples of suitable compounds are ethylene glycol, propylene glycol, glycerol and erythritol.

The poly(C₂-C₅ alkylene glycol) is preferably a polyethylene glycol or polypropylene glycol. The polyalkylene glycol may comprise any number of alkylene glycol units, for example having a molecular weight of at least 6000. Polyalkylene glycols may be liquid or solid at room temperature (20°C). It is preferred to use the solid form, particularly in the form of a free-flowing powder, for ease of handling and incorporation into the blend.

The polyol or poly(C₂-C₅ alkylene glycol) is generally present in the compositions of the present invention in an amount of from 1 to 50 wt%, preferably from 1 to 15 wt%, preferably 1.5 to 10 wt%, most preferably 2 to 6 wt%.

The polyol or poly(C₂-C₅ alkylene glycol) and the alginate may, for example, be present in the composition of the present invention in a weight ratio of from 2:1 to 1:25, preferably from 1:4 to 1:12.5.

The compositions of the present invention may also comprise further, optional components.

For example, the compositions of the present invention preferably comprise a source of divalent and/or trivalent metal ions. Such ions strengthen the raft formed in the stomach. Suitable metal ions are calcium and aluminium. The ions may be provided as part of the bicarbonate and/or carbonate, but may also comprise other anions if desired. For example, suitable sources of calcium ions are calcium carbonate, lactate, chloride, gluconate, phosphate, hydrogen phosphate, sulfate, tartrate or citrate, and suitable sources of aluminium ions are aluminium carbonate, lactate, glycinate or phosphate, aluminium magnesium carbonate, hydroxide or magaldrate, aluminium sodium carbonate hydroxide or aluminium sodium silicate. If used, the calcium ions are preferably present in an amount of from 8 to 800 parts, and the aluminium ions are preferably present in an amount of from 2 to 500 parts, per 500 parts by weight of alginate.

The compositions of the present invention may also comprise a preservative to prevent contamination and subsequent deterioration by micro-organisms. Examples of suitable preservatives are methyl, ethyl, propyl and butyl para-hydroxybenzoates and their salts, which are preferably used in combinations, for example methyl and propyl or ethyl and butyl. The compositions of the present invention do not need to include such a preservative, but if a preservative is present it may be used in an amount of, for example, up to 0.5 wt%, based on the total weight of the composition.

The compositions of the present invention may also comprise one or more colourings, sweetenings, flavourings, pH adjusting ingredients and fillers. When the compositions of the present invention are intended for use as sustained releasing compositions they will also comprise at least one active ingredient suitable for specific delivery to the stomach, such as a drug. Examples of suitable drugs are analgesics (e.g. sucacetaminophen, ibuprofen, naproxen, diclofenac, ketoprofen, choline salicylate, benzydamine, buprenorphine, hydrocortisone, betamethasone); decongestants (e.g. pseudoephedrine, phenylephrine, oxymetazoline, xylometazoline); cough suppressants (e.g. dextromethorphan, codeine, pholocodine); expectorants (e.g. guaiphenesin, n-acetylcysteine, bromhexine); antiseptics (e.g. triclosan, chloroxylenol, amylmetacresol, hexylresorcinol, dichlorobenzyl alcohol, benzyl alcohol); cardiovascular agents (e.g. glyceryl trinitrate); local anaesthetics (e.g. benzocaine, lignocaine); antacid agents (e.g. calcium carbonate, sodium bicarbonate, magnesium trisilicate, aluminium hydroxide, magaldrate,); antiulcer agents (e.g. carbenoxolone, sucralfate, cimetidine, ranitidine, nizatidine, famotidine, omeprazole, pantoprazole); antihistamines (e.g. loratidine, terfenadine, diphenhydramine, chlorphenhydramine, triprolidine, acrivastine); antinausea agents (e.g. prochlorperazine, sumatriptan); bowel regulatory agents (e.g. diphenoxylate, loperamide, sennosides); antifungal agents (e.g. clotrimazole); antimicrobial agents and antibiotics (e.g. fusafungine, tyrothricin).

It is also possible for the compositions of the present invention to comprise alginic acid, although this is not preferred since it could cause undesirable foaming in the mouth.

The compositions of the present invention may be in any solid form. For example they may be in the form of a tablet, such as a chewable tablet. They may also be in the form of a chewable gum, a confectionary such as a fudge or boiled sweet or in the form of particles or granules, for example free-flowing or packed in a capsule, for example a soft or hard gel capsule.

The composition of the present invention may be used in a method of treatment of the human or animal body by therapy, especially use in the treatment of reflux oesophagitis, gastritis, dyspepsia or peptic ulceration or for use as a sustained releasing or targeted delivery composition.

The composition of the present invention may be used in the manufacture of a medicament for the treatment of reflux oesophagitis, gastritis, dyspepsia or peptic ulceration or for use as a sustained releasing or targeted delivery composition.

The composition of the present invention may be used in a method of treating reflux oesophagitis, gastritis, dyspepsia or peptic ulceration or for sustained releasing or targeting a delivery composition, which comprises orally administering to a subject in need thereof or liable to need an effective amount of the composition.

The composition is generally administered in an amount of from 100 to 2000 mg alginate per dose.

The compositions of the present invention may be prepared by simply mixing the ingredients. It is especially preferred to mix the ingredients together in particulate form and then granulate or agglomerate the particles using a suitable granulating agent such as water, a C₂ to C₄ alcohol such as ethanol or isopropanol, or a mixture thereof. This is especially suitable when the PEG used is naturally a solid. It is also possible to granulate the remaining ingredients with PEG as a granulating agent when it is in liquid form. Additional granulating binders may also be used, for example povidone, a cellulose derivative such as HPMC or starch paste. A preferred starch paste uses water as the granulating solvent, and povidone is generally used with an ethanol or isopropanol solvent. We have surprisingly found that when a wet granulation is carried out, the amount of polyol or polyalkylene glycol can be reduced while retaining a satisfactory mouthfeel. A normal granulation process may need a weight ratio of polyol or polyalkylene glycol to aliginate of up to about 1:1. However, using a wet granulation process enables the weight ratio of polyol or polyalkylene glycol to alginate to be reduced to less than 0.25:1, especially less than 0.15:1, while retaining a satisfactory mouthfeel.

One or more of the components may be added after granulation. In particular the polyol or polyalkylene glycol may be added after granulation, although this is not preferred since an increased amount of this component may be required to achieve a suitable mouth feel. It is preferred to avoid the use of excessive amounts of polyol or polyalkylene glycol since the amount of this component which can be ingested may be limited by regulatory authorities.

In a preferred process for preparing the composition of the present invention, the alginate, carbonate and/or bicarbonate and polyol or polyalkylene glycol are granulated together, dried and screened prior to mixing in any further components. It is also possible, for example, to granulate only the alginate and the polyol or polyalkylene glycol prior to adding the remaining components.

The present invention is further described in the following Examples.

### EXAMPLES

### EXAMPLE 1

The following particulate components (each having a maximum particle size of 1mm) were mixed together in a high shear mixer for 1 minute:

| | |
|---|---|
| Sodium Alginate LFR 5/60 | 250g |
| Sodium bicarbonate | 133.5g |
| Calcium carbonate | 80g |
| PEG 20,000 | 30g |

The mixture was then granulated in a granulator using 75ml distilled, deionised water as a granulating agent.

The granules were then dried in a fluid bed drier at 40°C for 20 minutes and subsequently milled firstly through a 610µm screen and secondly through a 457µm screen using a Quadro Comill. The milled granulate was then blended with the following ingredients in a low shear tumble blender for 5 minutes:

| | |
|---|---|
| Mannitol | 522.75g |
| Crospovidone (dispersant) | 55g |
| Flavour 1 | 5.5g |
| Flavour 2 | 1.1g |
| Acesulfame K | 5.5g |
| Aspartame | 1.65g |

Finally, 15g magnesium stearate was added to the blender, and blending was continued for a further 2 minutes.

The granules were then compressed into tablets each containing 250mg or 500mg sodium alginate. The tablets were found to have a smooth, slightly chewy texture with no significant toothpacking or gummy residue.

### EXAMPLE 2

250 and 500 mg tablets were prepared following the procedure of Example 1 except for using the following components:

| | |
|---|---|
| Sodium Alginate LFR 5/60 | 250g |
| Sodium bicarbonate | 133.5g |
| Calcium carbonate | 80g |
| PEG 20,000 | 30g |
| Mannitol | 516.5g |
| Crospovidone | 55g |
| Flavour | 10g |
| Aspartame | 10g |
| Magnesium stearate | 15g |

### COMPARATIVE EXAMPLE 1 AND EXAMPLES 3 TO 7

A Comparative test was carried out to illustrate the beneficial effects of a polyalkylene glycol on the mouthfeel of a composition.

The following compositions were prepared:

| | EXAMPLES | | | | | |
|---|---|---|---|---|---|---|
| Ingredient (mg) | Comparative 1 | 3 | 4 | 5 | 6 | 7 |
| Protanal LFR 5/60 | 520 | 500 | 500 | 500 | 500 | 500 |
| NaHCO₃ | 177 | 170 | 170 | 170 | 170 | 170 |
| Mannitol | 1178 | 950 | 600 | 950 | 1125 | 300 |
| Mg-stearate | 31 | 30 | 30 | 30 | 30 | 30 |
| Kollidon 90F | 104 | - | - | - | - | - |
| PEG 20000 | - | - | 700 | 350 | 175 | 1000 |
| PEG 3000 | - | 350 | - | - | - | - |
| Total weight (mg) | 2010 | 2000 | 2000 | 2000 | 2000 | 2000 |
| PEG: Alginate | - | 1:1.43 | 1:0.71 | 1:1.43 | 1:2.86 | 1:0.5 |
| | | | | | | |
| | | | | | | |
| Mouthfeel | Very sticky in mouth + tooth-packing | Satisfactory for not sticking | Not sticking or tooth-packing. Slightly oily/creamy | Drying initially, slightly gritty. Then creamy. Not sticky | Creamy, not sticky | Quick dispersing. Very clean in the mouth i.e.not sticky or tooth-packing |

### COMPARATIVE EXAMPLE 2 AND EXAMPLE 8

Tablets produced by the method set out below were then evaluated for their mouthfeel.

| **Ingredient** | **Example** | |
|---|---|---|
| | **Comparative 2** | **8** |
| | **mg/tablet** | **mg/tablet** |
| Sodium alginate LFR5/60 | 250.00 | 250.00 |
| Sodium bicarbonate | 133.50 | 133.50 |
| Calcium carbonate | 80.00 | 80.00 |
| Mannitol | 607.75 | 432.75 |
| Polyethylene Glycol 20000 | 0.00 | 175.00 |
| Flavour 1 | 5.50 | 5.50 |
| Flavour 2 | 1.10 | 1.10 |
| Sweetener 1 | 5.50 | 5.50 |
| Sweetener 2 | 1.65 | 1.65 |
| Magnesium stearate | 15.00 | 15.00 |
| | | |
| Tablet weight | 1100mg | 1100mg |

### Processing

### Batch size produced 550g

1. Blend together ingredients except magnesium stearate for 5 minutes using a Turbula T2C tumble mixer.
2. Add the magnesium stearate and blend for a further 2 minutes.
3. Compress into tablets using the Riva Piccola tablet press fitted with 16mm FBE punches.

Tableting of the composition of Comparative Example 2 that did not contain PEG was poor, with evidence of lamination and capping.

The organoleptic properties of these tablets were assessed in the laboratory:

| Example | Toothpac king | Mouthfeel | Taste | After taste | Overall |
|---|---|---|---|---|---|
| Example 8 | Very slight | Drier, crisper. Tablet broke up quickly. | Pleasant, mint | None | OK - acceptable |
| Comparative 2 | Worst of all batches | Drying, cloying pasty, chewy & sticky | Pleasant, mint | None | Poor - unacceptable |

To check for raft formation properties and the appearance of the rafts, four crushed tablets (total 1g sodium alginate) were mixed with 20ml of water and poured into a 250ml beaker containing 150ml 0.1M HCl at 37°C. The ability to form a coherent foamy floating gel "raft" on the surface of the acid over 30 minutes was observed.

In both cases a floating raft was rapidly formed. This was continuous across the beaker surface and was resistant to rupture. No difference was observed between the two formulations.

Addition of PEG to the composition therefore has a significant effect on the mouthfeel of the product without affecting the ability to form a reflux-suppressing raft.

### EXAMPLE 9

Tablets containing the following components were prepared.

| | |
|---|---|
| Sodium aliginate LFR5/60 | 250.00 mg |
| Sodium bicarbonate | 133.50 mg |
| Calcium carbonate | 80.00 mg |
| Mannitol | 516.50 mg |
| Polyethylene Glycol 20000 | 30.00 mg |
| Crospovidone | 55.00 mg |
| Mint Flavour | 10.00 mg |
| Sweetener | 10.00 mg |
| Magnesium stearate | 15.00 mg |
| Tablet weight | 1100 mg |

### Process

1. Granulate components
   1.1 Add Sodium alginate LFR5/60, Sodium bicarbonate, Calcium carbonate, and Polyethylene Glycol 20000 to food processor Bowl (Magimix 3000 mixer fitted with large bowl).
   1.2. Turn on processor and blend the powders for 2 minutes.
   1.3 Granulate by spraying in water until a wet mass begins to form (approximately 70-110g water).
   1.4 Dry the granules in a fluid bed drier (Aeromatic Strea 1) at 40°C inlet air temperature.
   1.5 screen the dried granules using Quadro Comil mill fitted with a 457µm screen.
   1.6 sieve the granules through a 850µm sieve
2. Tableting mix
   2.1 Take the granules and the appropriate amount of the remaining ingredients except the magnesium stearate and tumble mix for 5 minutes.
   2.2 Add the magnesium stearate and tumble mix for a further 2 minutes.
3. Tableting
   3.1 Tablet the resulting tablet blend using the Riva Piccola bench top rotary tablet press fitted with 16mm FBE punches.

It was found that wet granulation of the components with the PEG enabled a reduced amount of PEG to be used as compared with the dry granulation of Example 8, while still retaining an acceptable mouthfeel.

### Example 10

Tablets were prepared from the following compositions, using the process described in Example 9.

| | |
|---|---|
| Sodium alginate LFR5/60 (sorbitol free) | 500.00mg |
| Potassium bicarbonate, medium granular | 100.00mg |
| Calcium carbonate | 100.00mg |
| Polyethylene glycol 20,000 | 60.00mg |
| Mannitol | 1260.00mg |
| Crospovidone | 110.00mg |
| Mint flavour | 20.00mg |
| Sweetener | 20.00mg |
| Magnesium stearate | 30.00mg |
| Total | 2200.00mg |

The tablets were produced to a weight of 500mg using 22mm flat bevel edge tooling.

These tablets gave satisfactory mouthfeel and raft formation and texture were at least as good as those of Example 9.

### Example 11

Example 10 was repeated but using the following compositions:

| | |
|---|---|
| Sodium Alginate LFR 5/60 | 250g |
| Sodium bicarbonate | 133.5g |
| Calcium carbonate | 80g |
| PEG 20,000 | 30g |
| Mannitol | 571.5g |
| Mint flavour | 10g |
| Sweetener | 10g |
| Magnesium stearate | 15g |

The tablets produced were considered to have a better mouthfeel than those of Example 10, even though they did not contain Crospovidone.

### Examples 12 to 17

Tablets were prepared from the following compositions:

| Ingredient | | | | | | |
|---|---|---|---|---|---|---|
| | 12 | 13 | 14 | 15 | 16 | 17 |
| | mg/tablet | mg/tablet | mg/tablet | mg/tablet | mg/tablet | mg/tablet |
| Sodium alginate LFR5/60 | 250.00 | 250.00 | 250.00 | 250.00 | 250.00 | 250.00 |
| Sodium bicarbonate | 50.00 | 250.00 | 50.00 | 133.00 | 133.00 | 50.00 |
| Calcium carbonate | 25.00 | 10.00 | 100.00 | 20.00 | 80.00 | 100.00 |
| Polyethylene Glycol 20000 | 175.00 | 175.00 | 175.00 | 175.00 | - | - |
| Polyethylene Glycol 3000 | - | - | - | - | 175.00 | 175.00 |
| Crospovidone | 1.10 | 1.10 | 1.10 | 1.10 | 1.10 | 1.10 |
| Flavour | 5.50 | 5.50 | 5.50 | 5.50 | 5.50 | 5.50 |
| Sweetener | 1.65 | 1.65 | 1.65 | 1.65 | 1.65 | 1.65 |
| Magnesium stearate | 15.00 | 15.00 | 15.00 | 15.00 | 15.00 | 15.00 |
| Mannitol | Qs | qs | qs | qs | qs | qs |
| Tablet weight | 1100mg | 1100mg | 1100mg | 1100mg | 1100mg | 1100mg |

### Example 18

### Tablets were prepared from the following compositions:

| Ingredient | mg/tablet |
|---|---|
| Sodium alginate | 500.00 |
| Potassium bicarbonate | 100.00 |
| Calcium carbonate | 100.00 |
| Polyethylene glycol 20,000 | 60.00 |
| Mannitol | 1370.00 |
| Flavouring | 20.00 |
| Sweetener | 20.00 |
| Magnesium Stearate | 30.00 |
| Total | 2200.00 |

## Claims

1. A solid, ingestible composition comprising:
a. an alginate;
b. a bicarbonate and/or carbonate; and
c. a C₂-C₅ polyol or poly(C₂-C₅ alkylene glycol) having a molecular weight of at least 6,000 and present in the composition in an amount of from 1 to 50 weight %.

2. A composition according to claim 1 wherein the polyalkylene glycol is polyethylene glycol (PEG).

3. A composition according to any one of the preceding claims wherein the alginate is sodium alginate.

4. A composition according to any one of the preceding claims wherein the bicarbonate is sodium bicarbonate.

5. A composition according to any one of the preceding claims wherein the carbonate is calcium carbonate.

6. A composition according to any one of the preceding claims which is in the form of a tablet.

7. A composition as defined in any one of the preceding claims for use in a method of treatment of the human or animal body by therapy.

8. A composition as defined in any one of claims 1 to 6 for use in the treatment of reflux oesophagitis, gastritis, dyspepsia or peptic ulceration or for use as a sustained releasing or targeted delivery composition.

9. Use of a composition as defined in any one of claims 1 to 6 in the manufacture of a medicament for the treatment of reflux oesophagitis, gastritis, dyspepsia or peptic ulceration or for use as a sustained releasing or targeted delivery composition.

10. A process for preparing a composition as defined in any one of claims 1 to 6 which comprises mixing together the alginate, the bicarbonate and/or carbonate and the polyol or polyalkylene glycol.

11. A process according to claim 10 wherein the components are granulated together in a wet granulation process.

12. A process according to claim 11 wherein the weight ratio of the polyol or polyalkylene glycol to the alginate is less than 0.25:1.

## Patentansprüche

1. Feste einnehmbare Zusammensetzung, umfassend
a) ein Alginat
b) ein Bicarbonat und/oder Carbonat und
c) ein C₂-C₅-Polyol oder Poly(C₂-C₅-alkylenglyol) mit einem Molekulargewicht von mindestens 6.000, das in der Zusammensetzung in einer Menge von 1 bis 50 Gew.-% vorliegt.

2. Zusammensetzung nach Anspruch 1, wobei das Polyalkylenglycol Polyethylenglycol (PEG) ist.

3. Zusammensetzung nach einem der vorangehenden Ansprüche, wobei das Alginat Natriumalginat ist.

4. Zusammensetzung nach einem der vorangehenden Ansprüche, wobei das Bicarbonat Natriumbicarbonat ist.

5. Zusammensetzung nach einem der vorangehenden Ansprüche, wobei das Carbonat Calciumcarbonat ist.

6. Zusammensetzung nach einem der vorangehenden Ansprüche, die in Form einer Tablette vorliegt.

7. Zusammensetzung wie definiert in einem der vorangehenden Ansprüche zur Verwendung in einem Verfahren zur therapeutischen Behandlung des menschlichen oder tierischen Körpers.

8. Zusammensetzung wie definiert in einem der Ansprüche 1 bis 6 zur Verwendung bei der Behandlung von Refluxösophagitis, Gastritis, Dyspepsie oder Ulkuskrankheit (PUD) oder zur Verwendung als eine Zusammensetzung mit Dauerfreisetzung oder gezielter Freisetzung.

9. Zusammensetzung wie definiert in einem der Ansprüche 1 bis 6 bei der Herstellung eines Medikaments zur Behandlung von Refluxösophagitis, Gastritis, Dyspepsie oder Ulkuskrankheit (PUD) oder zur Verwendung als eine Zusammensetzung mit Dauerfreisetzung oder gezielter Freisetzung.

10. Verfahren zur Herstellung einer Zusammensetzung wie definiert in einem der Ansprüche 1 bis 6, das das Zusammenmischen des Alginats, des Bicarbonats und/oder Carbonats und des Polyols oder Polyalkylenglycols umfasst.

11. Verfahren nach Anspruch 10, wobei die Bestandteile miteinander in einem Nassgranulierungsverfahren granuliert werden.

12. Verfahren nach Anspruch 11, wobei das Gewichtsverhältnis von Polyol oder Polyalkylenglycol zu Alginat weniger als 0,25:1 beträgt.

## Revendications

1. Composition solide pouvant être ingérée, comprenant :
a. un alginate ;
b. un bicarbonate et/ou carbonate ; et
c. un polyol en C₂ à C₅ ou poly(alkylèneglycol en C₂ à C₅) ayant un poids moléculaire d'au moins 6000 et présent dans la composition en une quantité de 1 à 50 % en poids.

2. Composition suivant la revendication 1, dans laquelle le polyalkylèneglycol est le polyéthylèneglycol (PEG).

3. Composition suivant l'une quelconque des revendications précédentes, dans laquelle l'alginate est l'alginate de sodium.

4. Composition suivant l'une quelconque des revendications précédentes, dans laquelle le bicarbonate est le bicarbonate de sodium.

5. Composition suivant l'une quelconque des revendications précédentes, dans laquelle le carbonate est le carbonate de calcium.

6. Composition suivant l'une quelconque des revendications précédentes, qui est sous forme d'un comprimé.

7. Composition telle que définie dans l'une quelconque des revendications précédentes, destinée à être utilisée dans une méthode de traitement de l'organisme de l'homme ou d'un animal par thérapie.

8. Composition telle que définie dans l'une quelconque des revendications 1 à 6, destinée à être utilisée dans le traitement de l'oesophagite peptique, de la gastrite, de la dyspepsie ou de l'ulcération peptique ou destinée à être utilisée comme composition à libération prolongée ou à administration ciblée.

9. Utilisation d'une composition telle que définie dans l'une quelconque des revendications 1 à 6 dans la production d'un médicament destiné au traitement de l'oesophagite peptique, de la gastrite, de la dyspepsie ou de l'ulcération peptique ou destinée à être utilisée comme composition à libération prolongée ou à administration ciblée.

10. Procédé pour la préparation d'une composition telle que définie dans l'une quelconque des revendications 1 à 6, qui comprend l'étape consistant à mélanger ensemble l'alginate, le bicarbonate et/ou carbonate et le polyol ou polyalkylèneglycol.

11. Procédé suivant la revendication 10, dans lequel les constituants sont granulés ensemble dans un procédé de granulation par voie humide.

12. Procédé suivant la revendication 11, dans lequel le rapport pondéral du polyol ou polyalkylèneglycol à l'alginate est inférieur à 0,25:1.
